# EUROPÄISCHE PATENTANMELDUNG

(11) **EP 1 752 172 A1**
(43) Veröffentlichungstag der Anmeldung: **14.02.2007**
(21) Anmeldenummer: 05017667.6
(22) Anmeldetag: 12.08.2005
(51) Int. Cl.: A61M 5/145, G01F 11/20

(54) **Antriebsvorrichtung für eine Infusionspumpe**

(71) Anmelder: F.HOFFMANN-LA ROCHE AG, 4070 Basel (CH); Roche Diagnostics GmbH, 68305 Mannheim (DE)
(72) Erfinder: Zwygart, Fritz, 3415 Hasle b. Burgdorf (CH); Liniger, Jürg, 3072 Ostermundigen (CH)
(74) Vertreter: Schwabe - Sandmair - Marx

(57) **Zusammenfassung**

Die Erfindung betrifft einen Antrieb für einen Verdrängungskörper (1) einer Infusionspumpe, wobei ein in einem Behältnis (2) bewegbarer Verdrängungskörper (1) von dem Antrieb (3,4,M) verschoben werden kann, wobei der Antrieb (3,4,M) zusammen mit dem Verdrängungskörper (1) bewegbar ist; einen Verdrängungskörper (1), dadurch gekennzeichnet, dass der Verdrängungskörper (1) an seiner Außenseite mindestens einen Teilabschnitt eines Außengewindes aufweist; ein System mit einem Behältnis (2) und mit einem bewegbaren Verdrängungskörper (1), gekennzeichnet durch ein Stopfenführungselement (2e,6), mit welchem der Verdrängungskörper (1) in das Behältnis (2) hineinbewegt werden kann oder an welchem sich der Verdrängungskörper (1) entlang bewegen kann sowie eine Infusionspumpe mit einem Druckerzeugungsmechanismus (7,8), um einen Verdrängungskörper (1) so mit einer Kraft zu beaufschlagen, dass eine in einem Behältnis (2) enthaltene Substanz in Richtung einer Substanzabgabeöffnung (2a) gedrückt wird und mit einem Dosierglied (9) zur Aufnahme mindestens eines vorgegebenen Volumens der Substanz (S) aus dem Behältnis (2) in einem Dosiervolumen (9a) des Dosiergliedes (9), so dass definierte Mengen der Substanz von dem Dosierglied (9) aufgenommen und zur Abgabe an einen Patienten bereitgestellt werden können.

## Beschreibung

Die vorliegende Erfindung bezieht sich auf eine Antriebsvorrichtung für eine Infusionspumpe, insbesondere auf einen Antrieb oder ein System zum Bewegen eines Verdrängungskörpers, wie zum Beispiel eines in einem Behältnis verschiebbaren Stopfens, durch dessen Bewegung eine zum Beispiel in einer beispielsweise aus Kunststoff gefertigten Ampulle oder Infusionspumpe enthaltene Substanz aus dem Behältnis ausgeschüttet werden kann.

Aus der DE 103 27 254 A1 ist eine modulare Infusionspumpe für die Verabreichung eines Produkts bekannt, wobei die für die Produktförderung erforderliche Kraft mittels eines Elektromotors erzeugt wird, dessen Antriebsgeschwindigkeit mittels eines Zahnradgetriebes untersetzt auf eine in dem Hauptgehäuse axial gerade geführte Kolbenstange übertragen wird, welche gegen einen in einem Reservoir aufgenommenen Kolben drückt, um ein in dem Reservoir enthaltenes Produkt zu verdrängen.

Aus der DE 102 40 165 A1 ist eine Vorrichtung zum dosierten Ausstoßen eines flüssigen Wirkstoffes für eine Infusionspumpe bekannt, wobei in einem Reservoir eine einen Wirkstoff enthaltene Flüssigkeit aufbewahrt wird und gegenüber einer Ausstoßöffnung des Reservoirs ein axial verschiebbarer Kolben angeordnet ist, welcher mittels einer Druckfeder permanent vorgespannt ist. An der Rückseite des Kolbens ist ein Seil als Haltmittel befestigt, dessen anderes Ende in einer Umfangsrille eines Sperrrades aufgewickelt ist und beim Vortrieb des Kolbens abgewickelt wird.

Es ist eine Aufgabe der vorliegenden Erfindung einen Pumpenantrieb vorzuschlagen, welcher platzsparend in einer Infusionspumpe eingebaut werden kann und zum Beispiel einen Stopfen in einem Reservoir oder einer Ampulle bewegen kann.

Eine allgemeine erfinderische Idee besteht darin, auf die üblicherweise vorgesehene Gewindestange zu verzichten und zum Beispiel den Ampullenkörper zur Kraftübertragung für den Stopfenantrieb zu verwenden, um so die Bauhöhe einer Infusionspumpe verkleinern zu können.

Gemäß einem Aspekt bezieht sich die Erfindung auf einen Antrieb für einen Verdrängungskörper, wie zum Beispiel einen in einer Ampulle oder einem Reservoir angeordneten oder einbringbaren und in der Ampulle oder dem Reservoir verschiebbaren bzw. bewegbaren Stopfen, wobei erfindungsgemäß der Antrieb, also zum Beispiel ein eine Drehbewegung erzeugender Motor mit einem zum Beispiel zusätzlich mit dem Motor gekoppelten Getriebe, unmittelbar an dem Verdrängungskörper anliegt oder befestigt ist, so dass der Antrieb, wie zum Beispiel der Motor mit zugehörigem Getriebe und Antriebselementen, zusammen mit dem Verdrängungskörper, wie zum Beispiel einem Stopfen, in ein Reservoir bzw. eine Ampulle hineinbewegt werden kann, um eine in der Ampulle oder dem Reservoir enthaltene Substanz zu verdrängen. Somit kann auf eine Kolbenstange verzichtet werden, welche in eine Ampulle eingeschoben werden muss und im aus der Ampulle ausgeschobenen Zustand zum Beispiel vor dem Einlegen einer vollen Ampulle in eine Infusionspumpe für eine Mindestbauhöhe der Infusionspumpe sorgt, welche mit einem erfindungsgemäßen Antrieb für einen Verdrängungskörper verringert werden kann.

Vorzugsweise weist der Antrieb mindestens ein und zum Beispiel zwei, drei oder mehr Eingriffelemente auf, welche mit dem Antriebselement, wie zum Beispiel dem Motor oder einem Getriebe, gekoppelt sind, um eine Bewegung des Motors, wie zum Beispiel eine Drehbewegung, auf das mindestens eine Eingriffelement, wie zum Beispiel ein Zahnrad oder eine Walze, welche in eine Innenseite des Reservoirs oder der Ampulle zum Beispiel mit spitzen Zähnen eingreifen kann, zu übertragen, um so eine Vorschubbewegung des zum Beispiel von dem Antrieb geschobenen oder gezogenen Verdrängungskörpers zu erzeugen. Als Eingriffelement können ein oder mehrere Räder oder Zahnräder vorgesehen sein, welche zum Beispiel gegen die Innenseite eines Behältnisses oder Reservoirs oder eine andere Abstützung gedrückt werden können und eine ausreichend hohe Reibung mit dem Behältnis haben oder zum Beispiel durch vorstehende Elemente oder Zähne in das Behältnis zum Beispiel an dessen Innenseite eingreifen, um eine auf das Eingriffselement übertragene Bewegung in eine Vorschub- oder Rückzugsbewegung des mit dem Antrieb gekoppelten Stopfens umzusetzen. Ebenso kann als Eingriffelement ein Schneckengetriebe bestehend aus einer oder mehreren Schnecken vorgesehen sein, wobei eine Schnecke zum Beispiel als zylinderförmiger Körper in Form einer Walze, als kegelförmiger Körper oder als ein anderer drehbarer Körper ausgebildet sein kann, welcher eine Außenfläche aufweist, die mit einer Anlagefläche an einem oder mehr als nur einem Punkt zum Beispiel über ein linienförmiges Teilstück in Berührung kommen kann und wobei an der Außenfläche des Körpers eine zum Beispiel spiralförmig umlaufende Nut vorgesehen sein kann, so dass beispielsweise ein relativ zu einem Reservoir verdrehsicher gelagerter Körper eine Eigendrehbewegung aufgrund des Eingriffes der mindestens einen schneckenförmig umlaufenden Nut in eine Vorschub- oder Rückzugbewegung in axialer Richtung des Reservoirs umsetzt.

Bevorzugt ist zusammen mit dem mindestens einen Eingriffelement ein Vorspannelement vorgesehen, welches auf das mindestens eine Eingriffelement so einwirken kann, dass dieses zum Beispiel gegen eine Anlagefläche, wie zum Beispiel die Innenseite eines Reservoirs oder einer Ampulle, gedrückt wird. Beispielsweise können Federn als Vorspannelement oder zwischen zwei oder mehr Eingriffselement einschiebbare kegelförmige Elemente, wie zum Beispiel eine Schnecke, verwendet werden, um das mindestens eine Eingriffelement aus einer Position, in welcher keine oder nur eine geringe Kopplung mit einer Anlagefläche, wie zum Beispiel einer Innenseite einer Ampulle, besteht, in eine Position mit ausreichender Kopplung oder einen Eingriff mit der Anlagefläche zu bringen, um durch eine Bewegung des mindestens einen Eingriffelementes eine Relativbewegung zur Anlagefläche und damit zum Beispiel relativ zu einem Reservoir oder einer Ampulle zu erzeugen, wodurch ein mit dem Eingriffselement gekoppelter Verdrängungskörper in dem Reservoir verschoben werden kann.

Weiterhin bezieht sich die Erfindung auf ein Behältnis, wie zum Beispiel eine Ampulle, welche aus Glas, Kunststoff oder einem anderen Material oder zum Beispiel dem Pumpengehäuse oder einem Teil davon gebildet wird, mit einem in dem Behältnis verschiebbaren oder bewegbaren Verdrängungskörper, wie zum Beispiel einem Stopfen, mit einem wie oben beschriebenen Antrieb.

Vorzugsweise weist das Behältnis eine Verdrehsicherung für den Antrieb und/oder den Verdrängungskörper auf, so dass dieser zum Beispiel in axialer Richtung des Behältnisses zum Beispiel durch eine Nut und/oder einen Steg als Führungselement relativ zum Behältnis verdrehgesichert geführt werden kann.

Weiterhin bezieht sich die Erfindung auf eine Infusionspumpe mit einem wie oben beschriebenen Behältnis.

Bei einem erfindungsgemäßen Verfahren ist oder wird ein Antriebs- oder Eingriffelement, wie zum Beispiel ein Zahnrad, eine Schnecke oder eine Walze mit einem Behältnis, bevorzugt einer Behältnisinnenseite, in Eingriff gebracht und durch Bewegung des Antriebselementes wird ein mit dem Antriebselement verbundener Verdrängungskörper in dem Behältnis bewegt.

Gemäß einem weiteren Aspekt bezieht sich die Erfindung auf einen Verdrängungskörper oder Stopfen für ein Behältnis, wie zum Beispiel eine Ampulle oder ein Reservoir, zur Verdrängung einer daraus abzugebenden medizinischen Substanz, wobei der Verdrängungskörper ein Gewinde oder Gewindeteilstück aufweist, welches in ein entsprechendes Gegengewinde des Behältnisses eingreifen und so zum Beispiel einen Teil des Behältnisses abdichten oder von einem anderen Teil des Behältnisses trennen kann, so dass der Verdrängungskörper in das Behältnis eingeschraubt werden kann, wobei durch die Einschraubbewegung des Verdrängungskörpers eine in dem Behältnis enthaltene Substanz aus dem Behältnis zum Beispiel durch eine Abgabeöffnung verdrängt wird. Das Gewinde am Verdrängungskörper kann ein umlaufender Steg oder mindestens ein Stegabschnitt sein, welcher in ein zum Beispiel durch eine umlaufende Nut gebildetes Innengewinde des Behältnisses eingreift. Ebenso kann das Innengewinde des Behältnisses durch einen umlaufenden Steg gebildet sein, welcher in mindestens eine korrespondierende Nut des Verdrängungskörpers eingreift, so dass der Verdrängungskörper durch den Gewindeeingriff mit dem Innengewinde des Behältnisses geführt wird und in dem Behältnis durch eine Drehung relativ zu diesem in axialer Richtung des Behältnisses bewegt werden kann.

An dem Verdrängungskörper und/oder dem Innengewinde des Behältnisses kann mindestens ein Dichtelement zum Beispiel bestehend aus einem Elastomer vorgesehen sein, um die Dichtwirkung an der Grenzfläche Verdrängungskörperaußenseite / Behältnisinnenseite zu erhöhen.

Weiterhin bezieht sich die Erfindung auf ein System mit einem wie oben beschriebenen Verdrängungskörper und einem Behältnis oder einer Ampulle mit einem Innengewinde.

Ein erfindungsgemäßer Verdrängungskörper kann mit einem Antrieb, wie zum Beispiel einem Motor, gekoppelt werden, welcher eine Drehbewegung relativ zum Behältnis auf den Verdrängungskörper überträgt, so dass der Verdrängungskörper über die Gewindeführung in das Behältnis bzw. in die Ampulle eingeschraubt werden kann und so eine Drehbewegung in eine Längsbewegung relativ zur Ampulle umsetzt, wodurch eine in der Ampulle enthaltene Substanz verdrängt werden kann.

Gemäß einem weiteren Aspekt bezieht sich die Erfindung auf ein Behältnis, wie zum Beispiel einen Teil einer Infusionspumpe oder eine Ampulle, mit einem in dem Behältnis verschiebbaren oder bewegbaren Verdrängungskörper, wie zum Beispiel einen Stopfen, wobei ein Stopfenführungselement durch zumindest einen Teil des Behältnisses hindurch oder in einem Behältnisinnenteil vorgesehen ist. Das Stopfenführungselement kann entweder mit dem Stopfen verbunden sein, wie zum Beispiel ein Zugelement, das z.B. an dem Stopfen befestigt ist und beispielsweise als Nylonfaden ausgebildet sein kann, um durch eine auf das Zugelement wirkende Kraft den Stopfen in das Behältnis hineinzuziehen, wobei gleichzeitig das Zugelement beispielsweise aus dem Behältnis durch eine Öffnung herausgezogen, in dem Behältnis aufgerollt oder umgelenkt und z.B. durch den Stopfen hindurch herausgezogen werden kann. Alternativ kann das Stopfenführungselement auch ein Abstützelement, wie zum Beispiel ein in dem Behältnis eingespritztes, eingegossenes, eingeklebtes oder anders eingebrachtes zum Beispiel stabförmiges Element sein, an welchem sich der Stopfen abstützen kann, um zum Beispiel durch am oder in der Nähe des Stopfens vorgesehene Klemm- oder Eingriffselemente den Stopfen entlang des zum Beispiel als starren Körper ausgebildeten Stopfenführungselements zu bewegen.

Dabei kann am oder im Stopfen ein wie oben beschriebenes Eingriffelement oder ein Linearaktuator vorgesehen sein, um den Stopfen schrittweise in das Behältnis einzuschieben. Der Linearaktuator kann zum Beispiel drei z.B. als Piezoelemente ausgebildete Aktuatoren aufweisen, wobei zwei der Aktuatoren oder Piezoelement so angesteuert werden können, dass diese selbst oder ein mit diesen verbundenes Druckelement gegen das Stopfenführungsteil, also z.B. einen Teil des Behältnisses, drücken können, um den Linearaktuator durch mindestens ein Piezoelement oder ein mit dem Piezoelement verbundenes Druckelement in einer Klemmverbindung oder kraftschlüssig mit dem bevorzugt starren Stopfenführungselement zu halten. Das dritte Piezoelement bzw. der dritte Aktuator ist zwischen den beiden anderen Piezoelementen vorgesehen und kann den Abstand zwischen diesen je nach angelegter Spannung verändern, das heißt verkleinern oder vergrößern. Werden die zur Erzeugung einer Klemm- oder Halteverbindung mit dem Stopfenführungselement vorgesehenen Aktuatoren oder Piezoelemente alternierend so angesteuert, dass wechselseitig immer eines davon nicht in Verbindung mit dem Stopfenführungselement ist oder keine Klemmwirkung mit diesem erzeugt, so kann, wenn das zwischen den Piezoelementen liegende und den Abstand dazwischen verändernde Piezoelement ebenfalls so alternierend angesteuert wird, dass der Abstand immer vergrößert wird wenn eines der beiden zur Klemmung dienenden Piezoelemente geöffnet und das andere geschlossen ist, eine Linearbewegung durch den Linearaktuator entlang des Stopfenführungselementes realisiert werden.

Weiterhin bezieht sich die Erfindung auf ein Verfahren zum Bewegen eines Verdrängungskörpers entlang eines Stopfenführungselementes, wobei ein erster Aktuator so angesteuert wird, dass eine Halteverbindung mit dem Stopfenführungselement hergestellt wird und ein zweiter Aktuator, welcher entlang des Stopfenführungselementes von dem ersten Aktuator entfernt angeordnet ist, so angesteuert wird, dass keine Haltewirkung mit dem Stopfenführungselement erzeugt wird und wobei ein zwischen den beiden Aktuatoren liegender weiterer Aktuator so angesteuert wird, dass die Entfernung zwischen den beiden Aktuatoren verändert wird, wonach der erste und zweite Aktuator so angesteuert werden, dass der zweite Aktuator in Halteeingriff gebracht wird und der erste Aktuator geöffnet wird und anschließend der zwischen den Aktuatoren liegende weitere Aktuator so angesteuert wird, dass er sich in die entgegengesetzte Richtung zur vorherigen Bewegungsrichtung bewegt.

Gemäß einem weiteren Aspekt bezieht sich die Erfindung auf eine Infusionspumpe mit einem Behältnis, wie zum Beispiel einer Ampulle oder einen Teil der Infusionspumpe, zur Aufnahme einer daraus abzugebenden Substanz, mit einer Substanzabgabeöffnung, wobei ein Druckerzeugungsmechanismus, wie zum Beispiel eine Feder, vorgesehen ist, um einen in dem Behältnis bewegbaren Verdrängungskörper so mit Druck zu beaufschlagen, dass die in dem Behältnis enthaltene Substanz aus der Substanzabgabeöffnung abgegeben werden kann. Erfindungsgemäß ist vor der Substanzabgabeöffnung ein Dosierglied mit mindestens einem Dosisaufnahmeelement zur Aufnahme einer oder mehrerer vorgegebener definierter Mengen oder Volumen aus dem Behältnis vorgesehen, wobei das Dosierglied so vor der Substanzabgabeöffnung angeordnet ist, dass die Einzelelemente des Dosiergliedes, in welche die vorgegebenen Volumina der abzugebenden Substanz aufgenommen werden können, von der Substanzabgabeöffnung wegbewegt und zu einer Abgabevorrichtung bewegt werden können, mit welcher die vorgegebenen Volumina aus dem Dosierglied abgegeben werden können. Somit können definierte Volumina oder Mengen einer Substanz aus dem Behältnis entnommen und an zum Beispiel eine Patientenleitung oder einen Patienten abgegeben werden, um eine genaue Dosierung der aus dem Behältnis abzugebenden Substanz zu ermöglichen.

Das Dosierglied kann eine oder mehrere Aufnahmeöffnungen zur Aufnahme einer definierten Menge der abzugebenden Substanz aufweisen, wie zum Beispiel definierte Volumen, die beispielsweise durch Taschen, Sacklöcher oder Durchgangslöcher des Dosiergliedes gebildet werden können, wobei im Falle eines Durchgangsloches dieses vorzugsweise während des Befüllvorganges einseitig abgeschlossen ist und nach dem Befüllen und einem optional möglichen Transport des Einfiillvolumens oder auch während der Befüllung der Verschluss des Durchgangsloches geöffnet werden kann, um das in dem Durchgangsloch enthaltene Volumen abzugeben. Vorzugsweise kann das Dosierglied als ein relativ zum Behältnis oder zur Ampulle bewegliches Element, wie zum Beispiel als drehbare Scheibe oder Trommel, ausgebildet sein, welche beispielsweise mehrere Durchgangslöcher enthält, die aus dem Behältnis befüllt werden können und welche im befüllten Zustand z.B. beidseitig durch an dem Dosierglied anliegende Dichtelemente z.B. aus einer hydrophoben Membrane zu einer Abgabevorrichtung, die beispielsweise in das Durchgangsloch oder ein zum Transport der Substanz verwendetes Volumen einführbar ist, bewegt werden können. Die Substanz ist mit der Abgabevorrichtung aus der Tasche oder dem Loch verdrängbar und zum Beispiel ausstoßbar, um die von der Tasche oder dem Durchgangsloch geförderte Substanz dosiert zum Beispiel an einen Patienten z.B. über eine an dem Dosiervolumen anliegende Leitung abzugeben.

Die Erfindung wird nachfolgend anhand von Ausführungsbeispielen beschrieben. Es zeigen:
- Figur 1: eine Querschnittsansicht eines Stopfenantriebs mittels Schneckengetriebe;
- Figur 2: eine Schnittansicht entlang der Linie A-A in Figur 1;
- Figur 3: eine Ausführungsform eines Schneckenantriebes für einen Stopfen;
- Figuren 3A und 3B: eine Schnittansicht entlang der Linie A-A in Figur 3 für den Schneckenantrieb in ausgekoppeltem und eingekoppeltem Zustand;
- Figur 3C: eine Schnittansicht einer Ampulle mit Verdrehsicherung;
- Figur 4: einen Längsschnitt durch eine Ampulle mit einem Schraubstopfenantrieb;
- Figur 4A: eine Schnittansicht entlang der Linie A-A in Figur 4;
- Figur 5A: eine Längsschnittansicht einer Ampulle mit Stopfenzugantrieb;
- Figur 5B: eine Längsschnittansicht einer Ampulle mit einem Piezo-Raupen-Stopfen-Antrieb;
- Figur 5C: eine vergrößerte Darstellung des Piezo-Antriebs gemäß Ausschnitt C aus Figur 5B;
- Figuren 6A bis 6C: die prinzipielle Darstellung des Bewegungsablaufes des Piezo-Antriebs;
- Figur 7: eine Querschnittsansicht eines entkoppelten volumetrischen Fördersystems; und
- Figur 7A: eine Draufsicht auf die Fördereinheit aus Figur 7.

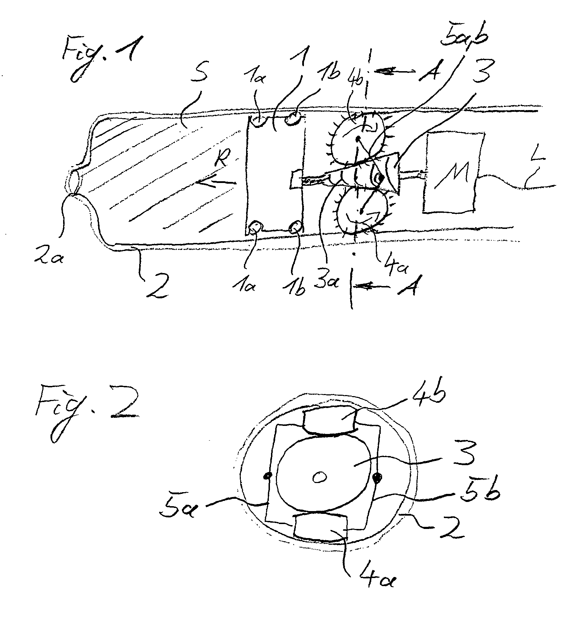

Figur 1 zeigt eine Querschnittsansicht einer Ausführungsform eines Schneckengetriebeantriebs für einen Stopfen 1 in einer Ampulle 2, in welcher eine Substanz S enthalten ist, welche durch eine Ampullenöffnung 2a abgegeben werden soll. Hierzu muss der Stopfen 1, welcher an seinem Umfang zur besseren Abdichtung gegen die Ampulle 2 zum Beispiel aus Gummi gefertigte um den Stopfen 1 umlaufende Dichtungsringe 1a und 1b aufweist, in die durch den Pfeil R dargestellte Richtung bewegt werden, um die Substanz S aus der Ampulle 2 zu verdrängen.

Mit dem Stopfen 1 z.B. drehbar verbunden oder an diesem anliegend ist eine Schnecke 3, welche von einem zum Beispiel verdrehsicher in der Ampulle 2 gelagerten Motor M angetrieben werden kann und eine konische oder Kegelform aufweist. Die Schnecke 3 hat ein umlaufendes Scheckengewinde 3a, in welches Zahnräder oder Walzen 4a und 4b eingreifen können, um eine Drehbewegung der Schnecke 3 in eine Abrollbewegung auf der Innenseite der Ampulle 2 umzusetzen. Mittels einer Vorspannvorrichtung 5a, 5b, welche zum Beispiel durch ein Federelement gebildet wird, welches zwei Federarme auseinanderdrückt, an welchen die Zahnräder 4a und 4b drehbar gelagert sind, kann sichergestellt werden, dass die Zahnräder 4a und 4b mit einer ausreichenden Kraft an der Innenseite der Ampulle 2 anliegen und zum Beispiel mit ihrer jeweiligen Zahnung in diese eingreifen, wobei die Ampulle 2 nach dem Gebrauch an ihrer Innenseite beschädigt oder zerstört werden kann.

Ist der Stopfen 1 soweit in die Ampulle 2 eingeschoben worden, dass keine weitere Substanz S mehr abgegeben werden soll oder kann, so kann der Motor M über die Steuer- und Stromversorgungsleitung L so angesteuert werden, dass der Schneckengetriebeantrieb mit oder ohne dem Stopfen 1 wieder aus der Ampulle 2 herausfährt, indem die Drehrichtung der mit dem Motor M verbundenen Schnecke 3 umgekehrt wird. Ebenso ist es auch möglich, dass der Motor M zum Beispiel an einem Draht (nicht gezeigt) oder an der Leitung L zusammen mit der mit dem Motor M gekoppelten Schnecke 3 und den zum Beispiel mit der Schnecke 3 gekoppelten Zahnrädern 4a und 4b und/oder zusammen mit dem Stopfen 1 aus der Ampulle 2 herausgezogen wird.

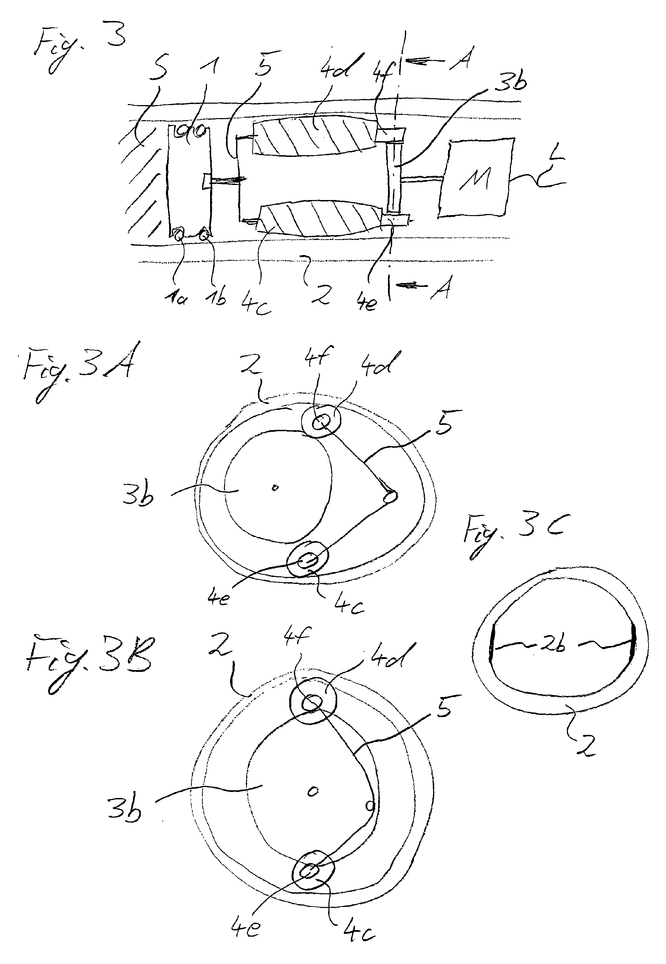

Wie aus Figur 2 ersichtlich, können die Zahnräder 4a und 4b zum Beispiel walzenförmig ausgebildet sein. Ebenso ist es möglich, alternativ anstatt der Zahnräder 4a und 4b nicht in die Ampulle 2 eingreifende Rollwalzen zu verwenden, welche von der Schnecke 3 angetrieben werden und eine Abrollbewegung auf die Innenseite der Ampulle 2 übertragen.

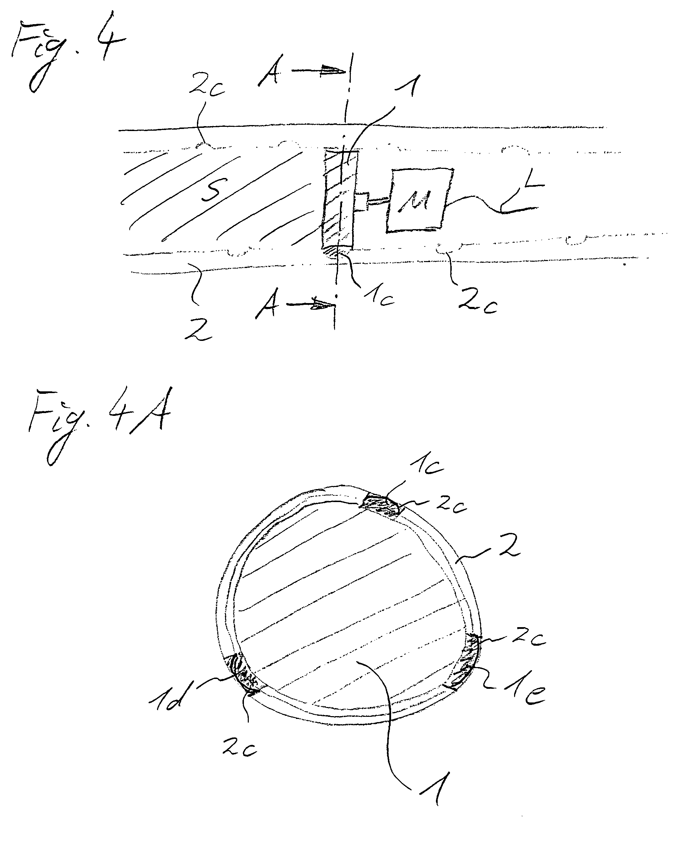

Figur 3 zeigt eine Ausführungsform eines Schneckenantriebs, wobei ein verdrehsicher gelagerter Motor M mit einer Versorgungs- und Steuerleitung L verbunden ist und eine Drehung auf ein Zahnrad 3b übertragen kann. Das Zahnrad 3b greift in Zahnräder 4e und 4f zweier oder mehrerer Schnecken 4c und 4d ein, welche so von dem Motor M angetrieben werden können und eine Eigendrehbewegung in Anlage an der Innenfläche der Ampulle 2 ausführen können. Die Schnecken 4c und 4d haben auf ihrer Außenseite gleichläufige zum Beispiel als umlaufende Nuten ausgebildete Schneckengewinde, welche bei einer Drehung der Schnecken 4c und 4d zu einer Bewegung der Schnecken 4c und 4d in axialer Richtung der Ampulle 2 führen. Die Schnecken 4c und 4d sind durch ein Vorspannelement 5 nach außen so vorgespannt, dass die Schnecken 4c und 4d immer an der Innenwand der Ampulle 2 zum Beispiel mit einer vorgegebenen definierten Kraft anliegen. Der durch die Schnecken 4c und 4d angetriebene Schneckenantrieb ist mit dem Stopfen 1 verbunden oder kann auf diesen drücken, um durch eine Bewegung des Stopfens 1 in Richtung auf die in der Ampulle 2 gelagerte Substanz S diese aus der Ampulle 2 zu verdrängen.

Figur 3A zeigt eine Ansicht entlang des Schnittes A-A in Figur 3, bei welcher das Antriebszahnrad 3b nicht im Eingriff mit den Zahnrädern 4e und 4f der Schnecken 4c und 4d ist. Wird das Zahnrad 3b seitlich verschoben, so gelangt es mit den Zahnrädern 4e und 4f der Schnecken 4c und 4d in Eingriff, so dass eine von dem verdrehsicher gelagerten Motor M erzeugte Drehbewegung des Zahnrades 3b auf die Schnecken 4c und 4d übertragen wird, wodurch sich die Schnecken 4c, 4d um ihre jeweiligen Mittelachsen drehen und durch die Anlage an der Innenseite der Ampulle 2 für eine axiale Bewegung des Schneckenantriebes sorgen.

Figur 3C zeigt eine Ausführungsform einer Verdrehsicherung für den Motor M, wobei auf der Innenseite der Ampulle 2 in Längsrichtung verlaufende Flächen oder Stege 2b vorgesehen sein können, welche mit Flächen oder in Nuten des Motors M oder eines Motorgehäuses oder eines anderen Elements des Schneckengetriebeantriebes wie in den Figuren 1 und 2 gezeigt oder des Schneckenantriebes gemäß Figur 3 einen Formschluss bilden oder eingreifen können, so dass der Motor M oder ein anderes Element des jeweiligen Stopfenantriebes verdrehsicher in der Ampulle 2 in axialer Richtung geführt werden kann.

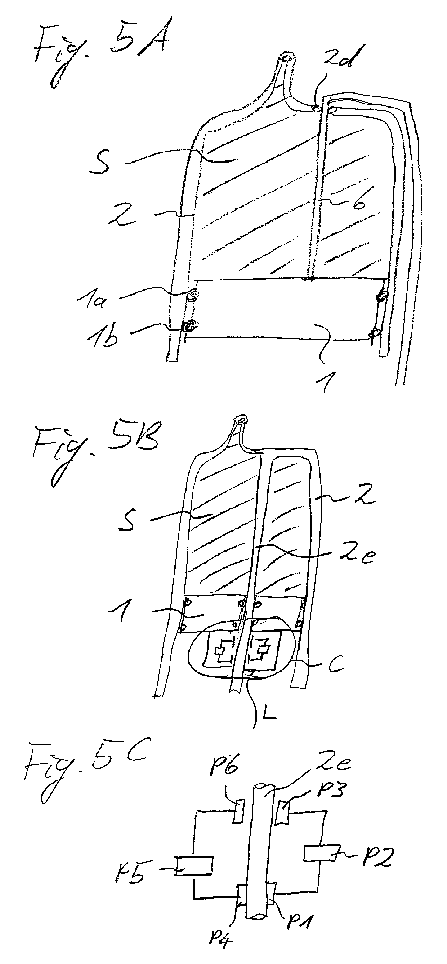

Figur 4 zeigt einen in ein oder mehrere umlaufende als Nuten ausgebildete Innengewinde 2c der Ampulle 2 eingreifenden Stopfen 1, welcher in Umfangsrichtung verteilt ein oder mehrere Dichtungselemente 1c, 1d und 1e aufweisen kann, die in jeweilige Innengewinde 2c der Ampulle 2 eingreifen können, wie in Figur 4A gezeigt, um eine sichere Abdichtung auch der in der Ampulle 2 vorgesehenen Gewindegänge 2c durch den Stopfen 1 zu gewährleisten. Der mit einem zum Beispiel verdrehsicher gelagerten Motor M verbundene Stopfen 1 kann durch den Motor M relativ zur Ampulle 2 gedreht werden und sich so in die Ampulle 2 durch die Führung der Innengewinde 2c einschrauben und so eine in der Ampulle 2 enthaltene Substanz S aus dieser verdrängen. Eine Drehung des Stopfens 1 führt somit über ein oder mehrere Gewinde 2c in der Ampulle 2 zu einer axialen oder Längsbewegung des Stopfens 1 in der Ampulle 2.

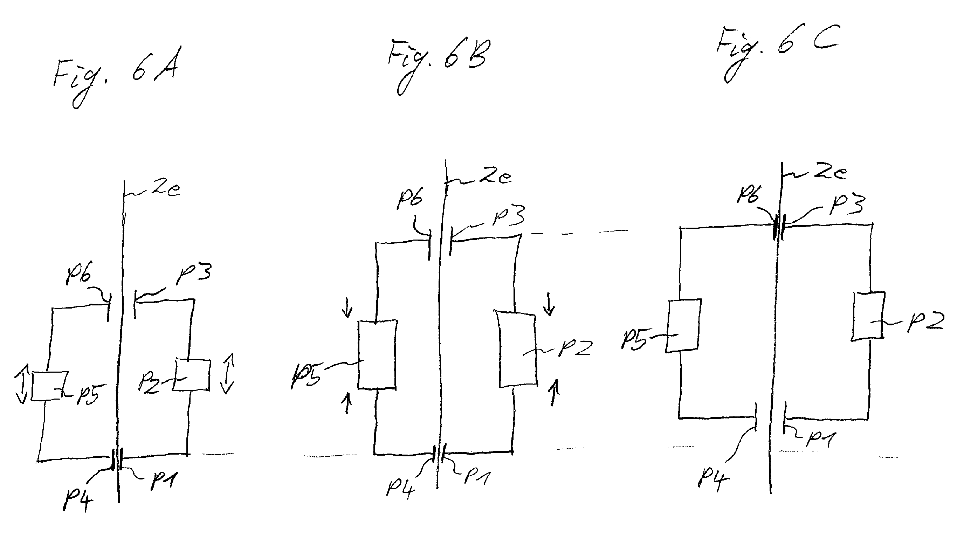

Figur 5A zeigt einen Zugantrieb für den Stopfen 1, welcher mit einem Faden oder Seil 6, wie zum Beispiel einem Nylonfaden mit bevorzugt zylindrischem Querschnitt, in die Ampulle 2 hineingezogen werden kann, wobei der Faden 6 durch eine mit Dichtungselementen 2d versehene Öffnung der Ampulle 2 aus dieser herausgeführt und zum Beispiel um die Ampulle 2 herumgelenkt werden kann, so dass kein unmittelbar hinter dem Stopfen 1 liegender Antrieb oder Motor benötigt wird, um den Stopfen 1 in die Ampulle 2 einzuschieben, wodurch die Bauhöhe der Infusionsvorrichtung verringert werden kann.

Figur 5B zeigt eine Ausführungsform eines Raupenantriebes, welcher an einem Stopfen 1 anliegt und diesen in eine Ampulle 2 einschieben oder aus dieser herausziehen kann. In der Ampulle 2 ist ein Stab oder zylinderförmiges Teil 2e eingesetzt oder eingespritzt, an welchem entlang sich der Raupenantrieb fortbewegen kann.

Der Raupenantrieb gemäß Einzelheit C ist vergrößert schematisch in Figur 5C dargestellt und umfasst in der Ausführungsform sechs Piezoelemente P1 bis P6, welche je nach angelegter Spannung ihre Länge bzw. Dicke verändern können. Mittels der Piezoelemente P1 und P4 kann eine Klemm- oder Halteverbindung an dem Stab 2e an einer ersten Stelle und mittels der Piezoelemente P3 und P6 an einer zweiten Stelle hergestellt oder gelöst werden, wobei der Abstand zwischen den Piezoelementen P1 und P3 durch das Piezoelement P2 und der Abstand zwischen den Piezoelementen P4 und P6 durch das Piezoelement P5 verändert werden kann. Vorzugsweise werden die Piezoelemente P 1 und P4 ebenso wie die Piezoelemente P2 und P5 oder die Piezoelemente P 3 und P6 paarweise durch gleiche Signale angesteuert.

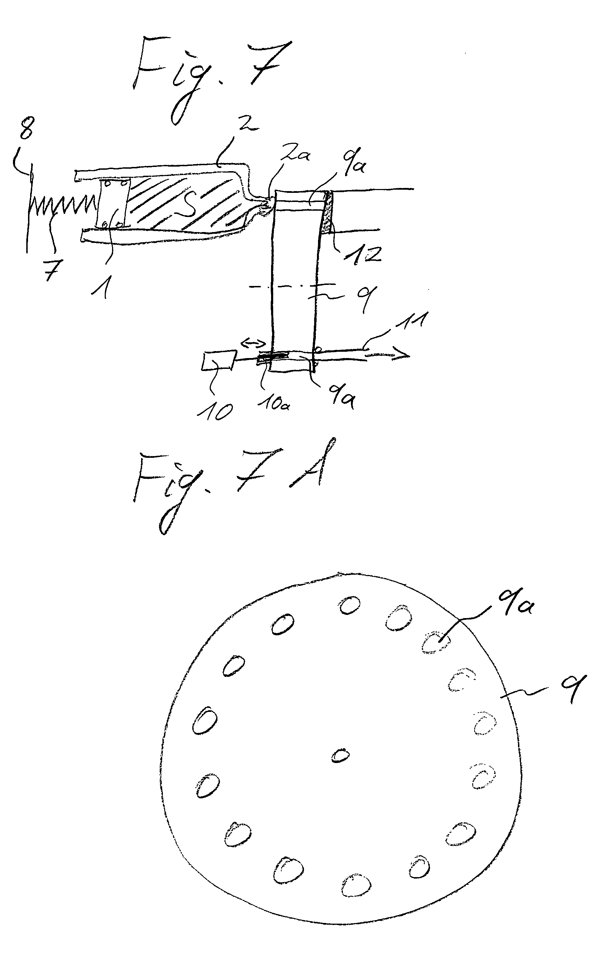

Die Figuren 6A bis 6C zeigen schematisch den Bewegungsablauf des Raupenantriebes in verschiedenen Bewegungsphasen. Dabei soll sich in Figur 6A der Raupenantrieb nach oben entlang des Stabes 2e bewegen. In Figur 6A ist eine Klemm- oder Halteverbindung an dem Stab 2e durch die Piezoelemente P 1 und P4 hergestellt und bei den Piezoelementen P3 und P6 gelöst worden. Die Piezoelement P2 und P5 werden so angesteuert, dass sich der Abstand zwischen den Piezoelementen P 1 und P3 bzw. zwischen den Piezoelementen P4 und P6 vergrößert, wie in Figur 6B gezeigt. Anschließend werden die Piezoelemente P3 und P6 so angesteuert, dass eine Klemm- bzw. Halteverbindung mit dem Stab 2e hergestellt werden kann und die Piezoelemente P1 und P4 werden so angesteuert, dass die Klemm- bzw. Halteverbindung mit dem Stab 2e gelöst wird, worauf die Piezoelement P2 und P5 so angesteuert werden, dass diese sich verkürzen und so der Abstand zwischen den Piezoelementen P 1 und P3 bzw. zwischen den Piezoelementen P4 und P6 verkürzt wird, wie in Figur 6C gezeigt. Anschließend kann wieder eine Klemm- bzw. Halteverbindung bei den Piezoelementen P 1 und P4 hergestellt und die Verbindung bei den Piezoelementen P3 und P6 gelöst werden, wie in Figur 6A gezeigt, worauf der Ablaufzyklus wiederholt werden kann, um den Raupenantrieb ein weiteres Stück nach oben entlang des Stabes 2e zu bewegen. Werden die Signale in umgekehrter Reihenfolge angelegt, so kann sich der Raupenantrieb nach unten bewegen.

Figur 7 zeigt eine Ausführungsform eines Antriebes zur entkoppelten volumetrischen Förderung einer Substanz S, welche aus einer Ampulle 2 abgegeben wird. Ein in die Ampulle 2 einschiebbarer Stopfen 1 ist durch eine Feder 7, welche sich zum Beispiel gegen das Gehäuse einer Infusionsvorrichtung 8 abstützt, vorgespannt und verdrängt eine in der Ampulle 2 enthaltene Substanz S aus dieser, wenn an einer Abgabeöffnung 2a der Ampulle 2 eine in einer Trommel 9 vorgesehene Durchgangsbohrung 9a vor der Öffnung 2a angeordnet ist. Die der an der Ausgabeöffnung 2a anliegenden Öffnung der Durchgangsbohrung 9a gegenüber liegende Öffnung liegt an einer hydrophoben Membrane 12 an, durch welche Luft, jedoch keine Substanz S, entweichen kann. Die Durchgangslöcher 9a der Trommel 9 können zum Beispiel ein Volumen von 10 µl fassen und können so sukzessive durch ein Drehen der Trommel 9 befüllt werden. Nach dem Befüllen werden die Durchgangslöcher 9a der Trommel entlang hydrophober Membranen 12 beidseitig abgeschlossen bis zu einem Ausstoßer 10 geführt, welcher zum Beispiel ein Piezoelement aufweisen kann, um ein Ausstoßelement 10a in die Durchgangsbohrung 9a einzuschieben und eine in der Durchgangsbohrung 9a enthaltene Substanz S aus der Durchgangsbohrung 9a in Richtung einer Abgabe- oder Patientenleitung 11 zu schieben.

Allgemein kann bei allen oben beschriebenen Ausführungsformen das Volumen in der Kammer oder Ampulle 2 unterteilt sein, um ein Ausstoßen in mehreren Schritten zu ermöglichen. Hierbei können zum Beispiel ein oder mehrere miteinander gekoppelte oder unabhängig voneinander bewegbare Stopfenelemente verwendet werden, welchen von einem, mehreren oder einer Kombination verschiedener oben beschriebener Antriebsmechanismen angetrieben oder bewegt werden können.

## Patentansprüche

1. Antrieb für einen Verdrängungskörper (1) einer Infusionspumpe zur dosierten Abgabe einer Substanz aus einem Behältnis (2), wobei ein in dem Behältnis (2) bewegbarer Verdrängungskörper (1) von dem Antrieb (3, 4, M) relativ zum Behältnis verschoben werden kann, **dadurch gekennzeichnet, dass** der Antrieb (3, 4, M) zusammen mit dem Verdrängungskörper (1) bewegbar ist.

2. Antrieb nach Anspruch 1, wobei ein Motor (M) des Antriebes (3, 4, M) zusammen mit dem Verdrängungskörper (1) in das Behältnis (2) hinein bewegbar ist.

3. Antrieb nach einem der vorhergehenden Ansprüche, wobei der Antrieb (3, 4, M) mindestens ein Eingriffelement (4a, 4b) aufweist, welches sich an der Innenseite des Behältnisses (2) abstützt oder zumindest teilweise in die Innenseite des Behältnisses (2) eindrückt, um für einen Vortrieb des Antriebes (3, 4, 1, M) zu sorgen.

4. Antrieb nach dem vorhergehenden Anspruch, wobei das mindestens eine Eingriffelement (4a, 4b) ein Zahnrad ist.

5. Antrieb nach Anspruch 3, wobei das mindestens eine Eingriffelement eine oder mehrere Schnecken (4a, 4d) sind, welche ein gleichläufiges oder gegenläufiges Gewinde aufweisen.

6. Antrieb nach einem der vorhergehenden Ansprüche mit einem Zahnrad (3b) oder einem Schneckengetriebe, insbesondere einer Schnecke (3), welche bevorzugt konisch oder kegelförmig ist und mindestens ein Eingriffelement (4) antreiben kann.

7. Antrieb nach einem der vorhergehenden Ansprüche mit einem Vorspannelement (5), um das mindestens eine Eingriffelement (4) in Richtung auf eine Wirkverbindung mit dem Behältnis (2), bevorzugt gegen die Innenseite des Behältnisses (2), vorzuspannen.

8. Antrieb nach einem der vorhergehenden Ansprüche, wobei ein Getriebe oder Getriebeelement (3) durch eine radiale und/oder axiale Bewegung relativ zum Behältnis (2) einen Motor (M) mit mindestens einem Eingriffelement (4) koppeln oder entkoppeln kann.

9. Infusionspumpe mit einem Antrieb nach einem der vorhergehenden Ansprüche und einem Behältnis (2) zur Aufnahme einer dosiert abzugebenden Substanz (S), wobei das Behältnis (2) eine Ampulle, insbesondere eine Kunststoff-Ampulle, ist oder von einem Pumpengehäuse gebildet wird.

10. Infusionspumpe nach dem vorhergehenden Anspruch, wobei in dem Behältnis (2) eine Verdrehsicherung, insbesondere mindestens eine bevorzugt in axialer Richtung verlaufende Nut oder Steg (2b) oder Flächen zum bevorzugt formschlüssigen Abschließen vorgesehen sind.

11. Verfahren zum Antreiben eines Verdrängungskörpers (1) in einem Behältnis, wobei ein Antriebselement (4a, 4b) des Antriebes mit dem Behältnis (2) in Eingriff gebracht wird oder ist und durch eine Bewegung des Antriebselementes (4a, 4b) ein mit dem Antrieb verbundener Verdrängungskörper (1) zusammen mit dem Antrieb in das Behältnis (2) bewegt wird.

12. Verdrängungskörper (1) zur Verdrängung einer durch Einbringen des Verdrängungskörpers (1) in ein Behältnis (2) aus dem Behältnis (2) dosiert abzugebenden Substanz (S), **dadurch gekennzeichnet, dass** der Verdrängungskörper (1) an seiner Außenseite mindestens einen Teilabschnitt eines Außengewindes aufweist.

13. Verdrängungskörper nach dem vorhergehenden Anspruch, wobei das mindestens eine Teil des Außengewindes des Verdrängungskörpers (1) als an der Außenseite umlaufender Steg oder Stegabschnitt ausgebildet ist.

14. Verdrängungskörper nach einem der zwei vorhergehenden Ansprüche, wobei mindestens ein Dichtelement (1c, 1d, 1e), bevorzugt ein Elastomer, an dem Außengewinde des Verdrängungskörpers (1) vorgesehen ist.

15. System zur dosierten Abgabe einer aus einem Behältnis (2) dosiert abzugebenden Substanz (S) mit einem Verdrängungskörper (1) nach einem der drei vorhergehenden Ansprüche, wobei das Behältnis (2) ein Innengewinde (2c) aufweist, in welcher der Verdrängungskörper (1) eingeschraubt oder eingedreht werden kann.

16. System mit einem Behältnis (2) für eine daraus dosiert abzugebende Substanz (S) mit einem in dem Behältnis (2) bewegbaren Verdrängungskörper (1), **gekennzeichnet durch** ein Stopfenführungselement (2e, 6), welches sich zumindest zum Teil in axialer Richtung innerhalb des Behältnisses (2) erstreckt und mit welchem der Verdrängungskörper (1) in das Behältnis (2) hineinbewegt werden kann oder an welchem sich der Verdrängungskörper (1) entlang bewegen kann.

17. System nach dem vorhergehenden Anspruch, wobei das Stopfenführungselement ein Zugelement (6), insbesondere ein Faden oder Seil, ist.

18. System nach einem der zwei vorhergehenden Ansprüche, wobei das Stopfenrührungselement eine Stange oder ein Stab (2e) ist, welche bzw. welcher sich in axialer Richtung durch zumindest einen Teil des Inneren des Behältnisses (2) erstreckt.

19. System nach einem der drei vorhergehenden Ansprüche, wobei an dem Verdrängungskörper (1) mindestens ein Dichtungselement (1a, 1b) angebracht ist, um eine Dichtung zwischen dem Verdrängungskörper (1) und dem Behältnis (2) und/oder dem Stopfenführungselement (2e) herzustellen.

20. Antriebselement für einen Verdrängungskörper (1), um den Verdrängungskörper (1) in ein Behältnis (2) einzuschieben und/oder aus dem Behältnis (2) herauszuziehen, wobei das Antriebselement mit dem Dichtungselement (1) verbunden ist oder an diesem anliegt und mindestens zwei Aktuatoren (P1, P4; P3, P6) aufweist, welche sich an dem Behältnis (2) abstützen oder an einem Teil davon festhalten oder festklemmen können und mindestens einen Aktuator (P2, P5) hat, mit welchem der Abstand zwischen dem ersten Aktuator (P1, P4) und dem zweiten Aktuator (P3, P6) verändert werden kann.

21. Antriebselement nach dem vorhergehenden Anspruch, wobei die Aktuatoren (P1-P6) durch Piezo-Elemente gebildet werden.

22. Verfahren zum Bewegen eines Verdrängungskörpers (1) in einem Behältnis (2), wobei der Verdrängungskörper (1) durch die Bewegung eines Antriebes in dem Behältnis (2) verschoben werden kann, bei welchem ein erstes Halteelement (P1, P4) eine Halteverbindung mit dem Behältnis (2) eingeht und ein zweites Halteelement (P3, P6) geöffnet wird, um keine Halteverbindung mit dem Behältnis (2) zu haben, der Abstand zwischen dem ersten Halteelement (P1, P4) und dem zweiten Halteelement (P3, P6) vergrößert wird, eine Halteverbindung des zweiten Halteelements (P3, P6) mit dem Behältnis (2) hergestellt wird und eine Halteverbindung des ersten Halteelements (P1, P4) gelöst wird und der Abstand zwischen dem ersten Halteelement (P1, P4) und dem zweiten Halteelement (P3, P6) verkleinert wird.

23. Infusionspumpe mit einem Behältnis (2) mit einer Substanzabgabeöffnung (2a), einem Druckerzeugungsmechanismus (7, 8), um einen Verdrängungskörper (1) so mit einer Kraft zu beaufschlagen, dass eine in dem Behältnis (2) enthaltene Substanz (S) in Richtung der Substanzabgabeöffnung (2a) gedrückt wird und mit einem Dosierglied (9) zur Aufnahme mindestens eines vorgegebenen Volumens der Substanz (S) aus dem Behältnis (2) in einem Dosiervolumen (9a) des Dosiergliedes (9), so dass definierte Mengen der Substanz (S) von dem Dosierglied (9) aufgenommen und zur Abgabe an einen Patienten bereitgestellt werden können.

24. Infusionspumpe nach dem vorhergehenden Anspruch, wobei der Verdrängungskörper ein Stopfen (1) und der Druckerzeugungsmechanismus eine Feder (7) ist.

25. Infusionspumpe nach einem der beiden vorhergehenden Ansprüche, wobei das Dosierglied (9) eine Mehrzahl von mindestens zwei vorgegebenen Aufnahmeelementen (9a) mit bevorzugt konstantem Volumen aufweist.

26. Infusionspumpe nach dem vorhergehenden Anspruch, wobei das Dosierglied so vor der Substanzabgabeöffnung (2a) des Behältnisses (2) bewegbar ist, dass die mindestens zwei Aufnahmeelemente (9a) zur Aufnahme der Substanz (S) aus dem Behältnis (2) abwechselnd vor die Substanzabgabeöffnung (2a) gebracht werden können.

27. Infusionspumpe nach einem der vier vorhergehenden Ansprüche, wobei das Dosierglied (9) eine Scheibe und/oder Trommel mit einer Mehrzahl von mindestens zwei Durchgangslöchern (9a) als Dosiervolumen ist.

28. Infusionspumpe nach einem der fünf vorhergehenden Ansprüche mit einer hydrophoben Membrane (12), welche mindestens eine Seite der Dosiervolumen oder Durchgangslöcher (9a) des Dosiergliedes (9) zumindest beim Zuführen der Substanz (S) aus dem Behältnis (2) abschließt.

29. Infusionspumpe nach einem der sechs vorhergehenden Ansprüche mit einer Abgabevorrichtung (10), welche ein Einschubelement (10a) aufweist, das in das Aufnahmeelement (9a) des Dosiergliedes (9) zur Verdrängung einer darin enthaltenen Substanz (S) eingeschoben werden kann.

30. Infusionspumpe nach dem vorhergehenden Anspruch, wobei die Abgabevorrichtung (10) ein Piezo-Element zum Einschieben des Verdrängungselementes (10a) in das Dosiervolumen (9a) aufweist.
